# FASCICULE DE BREVET EUROPEEN

(11) **EP 1 406 543 B1**
(45) Date de publication et mention de la délivrance du brevet: **19.12.2007**
(21) Numéro de dépôt: 02751279.7
(22) Date de dépôt: 20.06.2002
(51) Int. Cl.: A61B 17/04, A61B 17/06

(54) **ENSEMBLE COMPRENANT UNE ATTACHE DE FIXATION A USAGE MEDICAL ET UN DISPOSITIF DE MISE EN PLACE DE CETTE ATTACHE**
SATZ MIT MEDIZINISCHEM BEFESTIGUNGSELEMENT UND VORRICHTUNG ZUR ANORDNUNG DES BEFESTIGUNGSELEMENTS
KIT COMPRISING A MEDICAL FIXING ELEMENT AND A DEVICE FOR PLACING SAID FIXING ELEMENT

(30) Priorité: 21.06.2001 FR 0108204
(43) Date de publication de la demande: 14.04.2004
(73) Titulaire: SOFRADIM PRODUCTION, 01600 Trévoux (FR)
(72) Inventeur: THERIN, Michel, F-69004 Lyon (FR); ORY, François-Régis, F-69270 Fontaines St Martin (FR); BAILLY, Pierre, F-69002 Lyon (FR)
(74) Mandataire: Brédeville, Odile Marie
(86) Numéro de dépôt international: PCT/FR2002/002153
(87) Numéro de publication internationale: WO 2003/000140

(56) Documents cités:
- EP-A- 1 090 590
- FR-A- 2 774 277
- US-A- 4 932 962
- US-A- 4 935 028
- US-A- 5 085 661
- US-A- 5 437 680
- US-A- 6 156 044

## Description

La présente invention concerne un ensemble comprenant une attache de fixation à usage médical et une aiguille de mise en place de cette attache.

Il est connu de fixer une pièce ou un élément prothétique, notamment un renfort pariétal, à une paroi corporelle, notamment une paroi abdominale, au moyen d'au moins une attache de fixation en forme générale de "H", c'est-à-dire présentant deux barreaux d'arrêt reliés l'un à l'autre par une tige centrale souple. L'attache peut traverser la paroi, auquel cas l'un des barreaux d'arrêt prend appui contre la pièce prothétique tandis que l'autre barreau d'arrêt prend appui contre la paroi du côté opposé à la pièce prothétique ; l'attache peut aussi ne pas traverser la paroi, ledit autre barreau d'arrêt étant alors ancré à l'intérieur de cette paroi.

Une telle attache peut également être utilisée pour relier deux parois corporelles ou deux parties anatomiques.

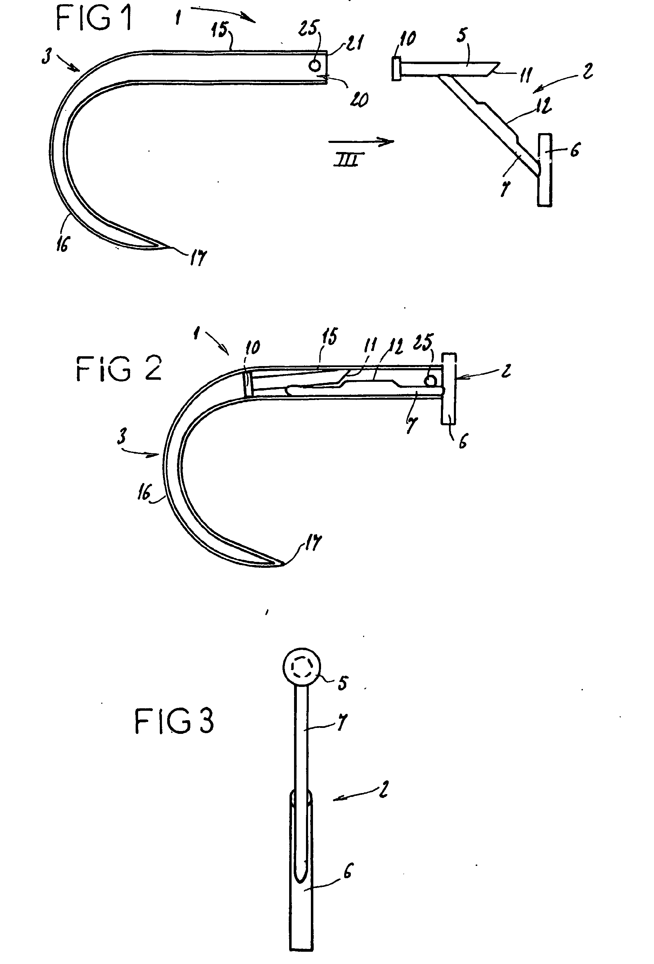

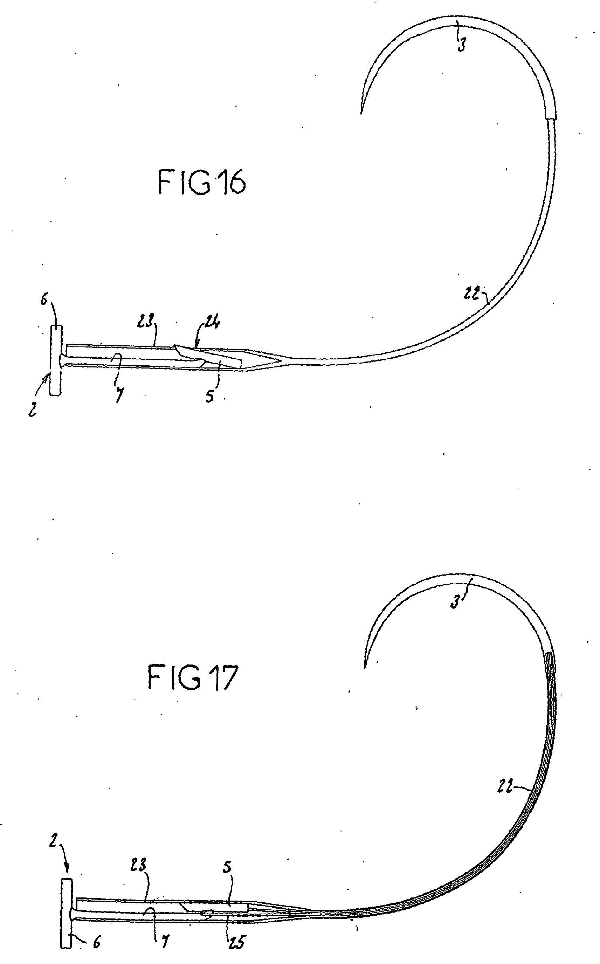

Les brevets américains n° 5 203 864 (cf. figure 17), n° 5 320 633 (cf. figure 1D) ou n° 4 696 300 (cf. figures. 2a, 2b) illustrent ce type d'attache et ces types de fixation.

Il existe différents types de dispositifs de mise en place de ces attaches.

Un premier type de dispositif comprend une aiguille creuse susceptible d'être engagée au travers de la ou des parois à équiper, qui présente une ouverture distale. Les barreaux d'arrêt des attaches peuvent être repliés parallèlement à la tige centrale de manière à ce que ces attaches puissent être engagées dans la cavité de l'aiguille. Le dispositif comprend également un poussoir proximal pouvant coulisser dans l'aiguille pour appuyer sur la ou les attaches contenues dans celle-ci afin de libérer sélectivement l'attache la plus distale.

Un second type de dispositif comprend également une aiguille creuse mais cette aiguille est fendue latéralement et ne reçoit que l'un des barreaux d'arrêt de chaque attache, la fente latérale permettant à ladite partie centrale et à l'autre barreau d'arrêt de chaque attache de dépasser sur le côté de l'aiguille.

Le document US 4,935,028 décrit un système de suture chirurgicale permettant la fermeture d'une plaie ou d'une incision, notamment dans une cornée, incluant une rondelle d'appui, une partie filliforme et une aiguille, la rondelle d'appui étant reliée à une extrémité de la partie filliforme et l'aiguille étant reliée à l'autre extrémité de cette partie filliforme. Après insertion dans la cornée, l'aiguille est séparée de la partie filliforme ou est coupée, l'extrémité correspondante adjacente de cette partie filliforme étant recourbée pour réaliser un ancrage, ou recevant une tête d'arrêt.

Avec le système selon ce document antérieur, la nécessité de couper l'aiguille par rapport à la partie filliforme puis de réaliser un ancrage ou un arrêt de l'extrémité de cette partie filliforme est une contrainte pratique certaine, rendant ce système incompatible avec la mise en oeuvre de techniques peu invasives telles que la coelioscopie. De plus, une mise en tension de l'attache, nécessaire pour assurer le maintien des deux parois à réunir étroitement plaquées l'une contre l'autre, est difficile à réaliser avec ce système. Un tel maintien est primordial dans certains cas, notamment pour la parfaite intégration tissulaire d'un renfort de paroi abdominale à la paroi abdominale traitée.

Le document EP 1 090 590 décrit une aiguille à fente latérale comprenant une attache libérable. Le système selon ce document n'est pas non plus à même de permettre la mise en tension précitée de l'attache.

La présente invention vise à remédier aux inconvénients de la technique antérieure.

Un objectif de l'invention est donc de fournir un ensemble de mise en place d'une attache avec tension, de manière à assurer le maintien des deux parois à réunir étroitement plaquées l'une contre l'autre.

Un autre objectif de l'invention est de fournir un ensemble particulièrement adapté au maintien d'un renfort de paroi abdominale intimement plaqué contre la paroi abdominale traitée, pour assurer une bonne intégration tissulaire de ce renfort à cette paroi abdominale.

Un objectif supplémentaire de l'invention est de fournir un ensemble parfaitement compatible avec la mise en oeuvre de techniques peu invasives telles que la coelioscopie.

Encore un autre objectif de l'invention est de fournir un ensemble particulièrement simple de structure et d'emploi, de manière à présenter des possibilités d'utilisation étendues.

Encore un autre objectif de l'invention est de fournir un ensemble pouvant être à usage unique.

L'ensemble concerné comprend, de manière connue en soi, une attache de fixation à usage médical et une aiguille de mise en place de cette attache, l'aiguille étant propre à être engagée au travers, ou dans l'épaisseur, de la ou des parois destinées à être équipées de l'attache, et cette attache présentant au moins une partie d'arrêt, propre à prendre appui contre une paroi, et une partie déformable filiforme reliée à ladite partie d'arrêt ; l'attache est élastiquement déformable au niveau de la liaison entre ladite partie d'arrêt et ladite partie déformable filiforme de telle sorte que la partie d'arrêt est mobile par rapport à la partie déformable filiforme entre une position de déformation élastique de ladite liaison, dans laquelle la partie d'arrêt et la partie déformable filiforme peuvent être substantiellement inscrites à l'intérieur de la section transversale de l'aiguille, et une position de non déformation, dans laquelle la partie d'arrêt est à même de prendre appui contre la ou les parois et d'assurer l'encrage de l'attache.

Selon l'invention, l'aiguille et l'attache comprennent des moyens de liaison amovible de la partie d'arrêt à la partie proximale de l'aiguille, cette liaison étant suffisamment solide pour que la partie d'arrêt reste solidaire de l'aiguille lors des manipulations de l'ensemble au moment de la saisie de cet ensemble, de l'introduction de celui-ci dans le corps du patient et de l'insertion de l'aiguille dans ladite paroi, mais étant libérable lorsqu'une traction est exercée sur l'aiguille, dans le sens d'insertion de l'aiguille, au-delà d'un seuil de force supérieur aux contraintes exercées sur l'attache par lesdites manipulations, la libération de l'attache permettant, par rappel élastique de ladite liaison, d'amener ladite partie d'arrêt en position de non déformation.

L'attache est ainsi libérée à la suite d'une traction exercée sur l'aiguille, dans le sens d'insertion de cette aiguille, ce qui permet une certaine mise en tension de l'attache par rapport à la ou aux parois à équiper. Cette mise en tension assure le maintien de deux parois à réunir étroitement plaquées l'une contre l'autre, en particulier un renfort de paroi abdominale et la paroi abdominale traitée.

L'aiguille a une structure particulièrement simple et un encombrement réduit, ce qui rend l'ensemble concerné très facile à utiliser, tant avec des techniques de coelioscopie qu'avec un abord direct.

L'ancrage peut être réalisé par un abord de la paroi à équiper au niveau de la face de cette paroi devant recevoir l'élément prothétique, ou peut être réalisé de manière transcutanée.

La simplicité de la structure de l'aiguille permet également que l'ensemble selon l'invention puisse être conçu de manière à être à usage unique, l'aiguille pouvant être jetée après utilisation. Une série de tels ensembles pourrait alors être conditionnée dans un emballage stérile, avantageusement conformé pour constituer, après ouverture, un présentoir facilitant les saisies successives de chaque ensemble. Cet emballage peut notamment être de type "blister".

Cette même simplicité de structure permet en outre de donner toutes formes appropriées à l'aiguille. En particulier, l'aiguille peut présenter une partie distale de forme courbe ou arquée, similaire à celle des aiguilles de suture.

Cette forme courbe ou arquée rend notamment possible de réaliser la fixation avec une attache en forme de "H" telle que précitée, dans laquelle les deux barreaux d'arrêt de l'attache sont situés sur la même face d'une paroi tissulaire, notamment au niveau d'un renfort pariétal, ladite partie filiforme déformable étant engagée dans l'épaisseur de la paroi selon une trajectoire recourbée qui correspond au trajet de l'aiguille dans cette paroi au moment de la mise en place de l'attache. Deux barreaux d'arrêt se trouvent ainsi positionnés contre l'élément prothétique, notamment un renfort pariétal, à implanter, ce qui permet un ancrage renforcé et performant de cet élément.

Selon une forme de réalisation possible de l'invention, l'aiguille présente une cavité intérieure débouchant dans sa partie proximale, et reçoit ladite partie d'arrêt de l'attache dans cette cavité.

Lesdits moyens de liaison amovible peuvent alors comprendre la paroi de l'aiguille délimitant ladite cavité et la partie d'arrêt de l'attache ; les dimensions de ladite cavité et de ladite partie d'arrêt sont alors déterminées de telle sorte qu'il existe des frottements entre cette partie d'arrêt et cette paroi de l'aiguille.

Lesdits moyens de liaison amovible peuvent également comprendre, cumulativement ou alternativement à la liaison par frottements citée ci-dessus, un ou plusieurs bossages faisant saillie à l'intérieur de la cavité, propres à constituer un "point dur" devant être franchi par ladite partie d'arrêt pour permettre de libérer l'attache.

Chaque bossage peut notamment être formé par un embouti aménagé dans la zone d'extrémité proximale de l'aiguille.

Selon une forme de réalisation de l'invention dans ce cas,
- l'aiguille présente une cavité proximale ayant un diamètre constant sur une portion proximale destinée à recevoir l'attache et présentant une réduction progressive de ce diamètre sur une portion distale ;
- l'attache comprend un barreau distal, un barreau proximal et une partie intermédiaire reliant ces barreaux l'un à l'autre ; le barreau distal présente une collerette dont le diamètre est légèrement inférieur au diamètre de ladite portion proximale de la cavité, de sorte que cette collerette peut être introduite et peut coulisser dans cette portion proximale jusqu'au niveau de la portion distale de cette cavité, dans laquelle cette collerette se coince ; la longueur de la partie intermédiaire est telle que le barreau proximal vient au contact de l'extrémité proximale de l'aiguille lorsque la collerette se trouve en un emplacement produisant un coincement adéquat de cette collerette.

La venue en butée du barreau proximal contre l'extrémité proximale de l'aiguille permet ainsi de définir un enfoncement déterminé de la collerette dans la cavité de l'aiguille, correspondant au coincement idéal à obtenir. Ce coincement idéal est suffisant pour résister aux frottements précités subis par l'attache mais est insuffisant pour risquer d'engendrer une détérioration de l'attache lorsqu'il est souhaité de supprimer la liaison entre l'aiguille et l'attache.

Selon une autre forme de réalisation possible de l'invention, l'ensemble comprend une partie filiforme souple reliée à l'extrémité proximale de l'aiguille et à un manchon recevant ladite partie d'arrêt de l'attache.

La partie filiforme souple augmente ainsi les possibilités de mobilité de l'aiguille par rapport à l'attache, ce qui facilite la manipulation de l'aiguille en vu de son insertion au travers de la ou des parois à équiper, et permet d'utiliser une aiguille de suture classique. Cette même partie filiforme souple permet que l'attache soit située à distance de l'aiguille lors des manipulations de cette aiguille, de manière à éliminer tout risque de séparation intempestive de l'attache et de l'aiguille lors de ces manipulations.

Un moyen simple de réaliser l'ensemble selon l'invention dans ce cas est d'utiliser un tube en matériau thermorétractable, notamment en polytétrafluoroéthylène (PTFE), et de réaliser une thermorétraction de ce matériau sur la majeure partie de ce tube, pour constituer ladite partie filiforme souple, sans réaliser de thermorétraction sur le reste de ce tube, destiné à constituer ledit manchon.

Dans cette forme de réalisation de l'invention, lesdits moyens de liaison amovible peuvent comprendre les dimensions respectives du manchon et de l'attache, ces dimensions étant telles qu'il existe des frottements entre l'attache et le manchon lorsque cette attache est insérée dans ce manchon. Ces dimensions peuvent notamment résulter du choix judicieux du diamètre du tube en matériau thermorétractable.

Ces mêmes moyens de liaison amovible peuvent comprendre une ouverture latérale aménagée dans le manchon, dans laquelle est engagée amoviblement une portion de l'attache, en particulier une extrémité que présente ladite partie d'arrêt. La séparation de l'attache et du manchon peut alors être réalisée par déchirure de la paroi du manchon à partir de cette fenêtre.

Lesdits moyens de liaison amovible peuvent également comprendre un fil présent dans le manchon, engagé amoviblement autour d'une partie de l'attache. Ce fil peut notamment former une boucle autour d'une partie de l'attache et être ruptible. Dans le cas précité de l'utilisation d'un tube en matériau thermorétractable, ce fil est simplement engagé dans le tube avant de réaliser la thermorétraction.

Selon encore une autre forme de réalisation possible de l'invention, l'aiguille et ladite partie d'arrêt de l'attache sont reliées l'une à l'autre par un pont de matière dimensionné de façon à se rompre au-delà dudit seuil de force. Dans ce cas, l'aiguille et l'attache peuvent être moulées en une seule et même pièce d'un matériau approprié.

Ladite partie d'arrêt de l'attache peut présenter la forme d'un barreau relié à ladite partie filiforme déformable sensiblement au niveau de sa zone médiane.

L'attache peut comprendre une partie d'arrêt unique ou peut comprendre deux parties d'arrêt reliées l'une à l'autre par ladite partie filiforme déformable.

Ces deux parties d'arrêt permettent l'ancrage renforcé et performant précité.

Les deux parties d'arrêt peuvent notamment être en forme de barreaux. La partie d'arrêt proximale, étant donné qu'elle n'a pas à être introduite dans l'aiguille, peut avoir toute forme appropriée autre qu'une forme de barreau, susceptible d'améliorer sa capacité à former un arrêt. Elle peut par exemple avoir une forme en sphère, en disque ou en anneau.

Par ailleurs, grâce à l'invention, il n'existe pas de limitations dans la longueur qu'il est possible de donner à ladite partie filiforme déformable ; cette partie filiforme déformable peut notamment être formée par un fil propre à être utilisé pour réaliser une suture, les parties d'arrêt dispensant alors de faire des noeuds ; elle peut également présenter une longueur telle que plusieurs passages successifs de l'aiguille au travers de la ou des parois à équiper sont possibles. Dans ce deuxième cas, la partie filiforme déformable peut présenter une relative rigidité lui conférant, lorsqu'elle est insérée dans la ou les parois selon une trajectoire courbe ou ondulée, une capacité de rappel élastique vers sa forme rectiligne. Cette capacité de rappel assure le maintien d'une pièce prothétique plaquée contre la paroi ayant reçu l'attache. Alternativement, dans le même but, la partie filiforme déformable peut présenter une élasticité longitudinale.

L'attache peut en outre être en un matériau biorésorbable.

Pour sa bonne compréhension, l'invention est à nouveau décrite ci-dessous en référence au dessin schématique annexé représentant, à titre d'exemples non limitatifs, plusieurs formes de réalisation possibles de l'ensemble qu'elle concerne et de différentes variantes de réalisation possibles de l'attache que comprend cet ensemble.
La figure 1 est une vue de côté, avant montage, de l'aiguille et de l'attache que comprend ledit ensemble, selon une première forme de réalisation ;
la figure 2 en est une vue similaire à la figure 1, après montage ;
la figure 3 est une vue de l'attache selon la flèche III de la figure 1 ;
les figures 4 à 7 sont des vues de côté dudit ensemble et d'une paroi tissulaire dans laquelle l'attache est destinée à être mise en place, montrant respectivement quatre étapes successives de mise en place de cette attache dans cette paroi ;
les figures 8 à 11 sont des vues de côté dudit ensemble selon une variante et d'une paroi tissulaire dans laquelle l'attache est destinée à être mise en place, montrant respectivement quatre étapes successives de mise en place de cette attache dans cette paroi ;
la figure 12 est une vue est une vue dudit ensemble, similaire à la figure 2, selon une autre forme de réalisation de l'attache ;
les figures 13 à 15 sont des vues de côté dudit ensemble et d'une paroi tissulaire dans laquelle l'attache est destinée à être mise en place, montrant trois étapes successives de mise en place de cette attache dans cette paroi ;
la figure 16 est une vue est une vue dudit ensemble, similaire à la figure 12, selon une variante de réalisation ;
la figure 17 est une vue est une vue dudit ensemble, similaire à la figure 12, selon une autre variante de réalisation ;
la figure 18 est une vue dudit ensemble, similaire à la figure 2, selon une variante de réalisation de l'attache;
la figure 19 est une vue dudit ensemble, similaire à la figure 2, selon une autre variante de réalisation de l'attache, et
la figure 20 est une vue dudit ensemble, similaire à la figure 2, selon une autre forme de réalisation.

Par simplification, les parties ou éléments de la première forme de réalisation, qui se retrouvent dans les autres formes de réalisation ou variantes, seront désignés par les mêmes références numériques et ne seront pas à nouveau décrits.

Les figures 1 et 2 représentent un ensemble 1 comprenant une attache de fixation 2 à usage médical et une aiguille 3 de mise en place de cette attache 2.

L'attache 2 est moulée en une pièce dans une matière synthétique déformable, pouvant être biorésorbable. Elle comprend un premier barreau d'arrêt 5, un deuxième barreau d'arrêt 6 et une partie intermédiaire filiforme 7 reliant ces barreaux 5, 6 l'un à l'autre.

L'attache 2 est représentée sur la figure 1 dans sa forme neutre, c'est-à-dire en l'absence de toute déformation. Selon cette forme, les barreaux 5, 6 forment un angle de sensiblement 90 degrés entre eux et la partie intermédiaire 7 est orientée par rapport aux barreaux 5, 6 de manière sensiblement perpendiculaire à la bissectrice de l'angle formé par ces barreaux 5, 6.

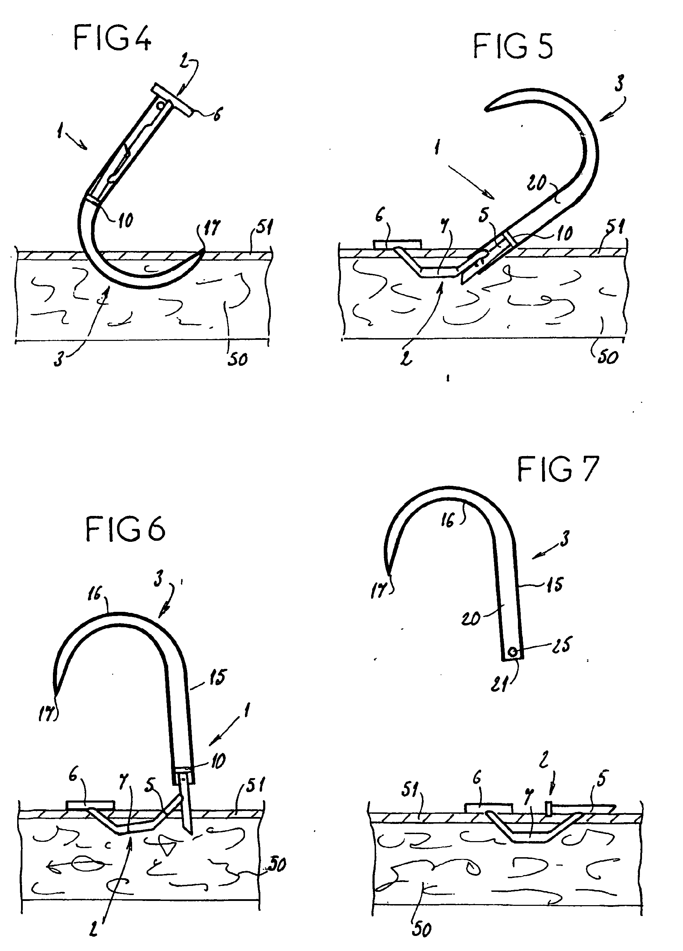

En référence à la figure 2, il apparaît que la déformabilité de la partie intermédiaire 7 permet de replier le barreau 5 dans le prolongement de cette partie intermédiaire 7 ; en référence à la figure 7, il apparaît que cette même déformabilité permet que les barreaux 5, 6 puissent être placés sensiblement dans un même plan après mise en place de l'attache 2 sur la paroi tissulaire 50.

Le barreau 5 présente un diamètre supérieur à celui de la partie intermédiaire 7, ce qui lui confère une rigidité longitudinale supérieure à celle de cette partie 7, et est relié à cette dernière au niveau de sa portion médiane. A une extrémité distale (considérée par rapport au sens d'engagement de l'aiguille dans la paroi 50), il comprend une collerette transversale 10, d'un diamètre déterminé qui sera explicité plus loin. A son extrémité proximale, il présente un biseau 11. Ainsi que le montre la figure 2, ce biseau 11 forme un dégagement permettant de replier le barreau 5 contre la partie 7 nonobstant un renflement médian 12 que présente cette partie 7.

Le barreau 6 présente également un diamètre supérieur à celui de la partie intermédiaire 7, de manière à avoir une rigidité longitudinale supérieure à celle de cette partie 7, et est également relié à cette dernière au niveau de sa partie médiane. Il présente une forme purement cylindrique.

La partie intermédiaire 7, outre sa déformabilité, son orientation et son renflement 12 précités, présente une longueur déterminée, qui sera explicitée ci-dessous.

L'aiguille 3 est en acier inoxydable. Elle présente une partie proximale rectiligne 15, de structure tubulaire, et une partie distale arquée 16. Le diamètre de l'aiguille 3 se réduit progressivement en direction de l'extrémité distale 17 de cette aiguille, cette réduction de diamètre débutant au niveau de l'extrémité distale de la partie proximale 15.

La partie proximale 15 définit ainsi une cavité 20 présentant un diamètre constant sur une large portion proximale et présentant une réduction progressive de ce diamètre sur une portion distale. Ainsi que le montre la figure 2, la portion proximale de la cavité 20 est propre à recevoir le barreau 5 et la partie intermédiaire 7 lorsque ce barreau 5 est replié dans le prolongement de cette partie intermédiaire 7; le diamètre de la collerette 10 est légèrement inférieur au diamètre de la portion proximale de la cavité 20, de sorte que cette collerette 10 peut être introduite et peut coulisser dans ladite portion proximale de cette cavité 20 jusqu'au niveau de la portion distale de cette cavité 20, dans laquelle cette collerette 10 se coince.

Ainsi que le montre également la figure 2, la longueur de la partie intermédiaire 7 est telle que le barreau 6 vient au contact de l'extrémité proximale 21 de l'aiguille 3 lorsque la collerette 10 se trouve en un emplacement produisant un coincement adéquat de cette collerette 10 dans la cavité 20. Ce coincement adéquat est tel qu'il produit une liaison amovible de l'attache 2 à la partie proximale 15 de l'aiguille 3. Cette liaison est suffisamment solide pour que l'attache 2 reste solidaire de l'aiguille 3 lors des manipulations de l'ensemble 1 au moment de la saisie de cet ensemble 1, de l'introduction de celui-ci dans le corps du patient et de l'insertion de l'aiguille 3 dans la paroi 50 à équiper, mais est libérable lorsque l'attache 2 vient prendre appui contre la paroi 50 et qu'une traction manuelle est exercée par le praticien sur l'aiguille 3.

L'aiguille 3 présente en outre, dans sa partie d'extrémité proximale, un bossage 25 faisant saillie vers l'intérieur de la cavité 20, formé par un embouti dans la paroi de l'aiguille 3. Ce bossage 25 est propre à constituer un "point dur" devant être franchi par la collerette 10 pour permettre de séparer l'attache 2 de l'aiguille 3.

En pratique, ainsi que le montrent les figures 4 à 7, un renfort pariétal 51 est mis en place le long de la paroi 50 à renforcer puis l'ensemble 1 est amené près de cette paroi 50.

La portion arquée 16 de l'aiguille 3 est alors insérée partiellement au travers du renfort 51 et de la paroi 50 (figure 4), selon un mouvement circulaire sensiblement centré sur le centre générant cette partie arquée 16, selon le geste classique d'utilisation d'une aiguille courbe de suture.

La portion distale de la partie arquée 16 qui ressort de la paroi 50 et du renfort 51 suite à cette insertion est ensuite saisie et est tirée jusqu'à engager la partie proximale 15 de l'aiguille dans la paroi 50 (figure 5). Cet engagement amène le barreau 6 à porter contre le renfort 51 et à décoincer la collerette 10, autorisant ainsi la sortie de la partie intermédiaire 7 hors de la cavité 20 au fur et à mesure du coulissement de la partie 15 dans la paroi 50.

L'aiguille 3 est ensuite progressivement extraite de la paroi 50 et du renfort 51, par traction exercée sur elle selon une direction parallèle à celle qu'occupe la partie 15 sur la figure 5, jusqu'à extraction complète (figure 6). La collerette 10 rencontre alors le bossage 25.

Une traction est alors exercée sur l'aiguille 3 selon une direction parallèle à celle qu'occupe la partie 15 sur la figure 6, pour permettre à la collerette 10 de franchir le bossage 25. Cette traction permet d'extraire le barreau 5 de la cavité 20 en l'écartant du renfort 51, de sorte que ce barreau 5 vient ensuite, par rappel élastique de la partie intermédiaire 7, se plaquer contre le renfort 51 et plaquer ce renfort 51 contre la paroi 50 (figure 7). L'aiguille 3 est ensuite retirée et jetée.

Les opérations décrites ci-dessus sont reproduites autant de fois que nécessaire en différents emplacements du renfort 51 pour assurer la parfaite fixation de ce renfort 51 à la paroi 50. Un nouvel ensemble 1 est utilisé pour chaque mise en place d'une attache 2.

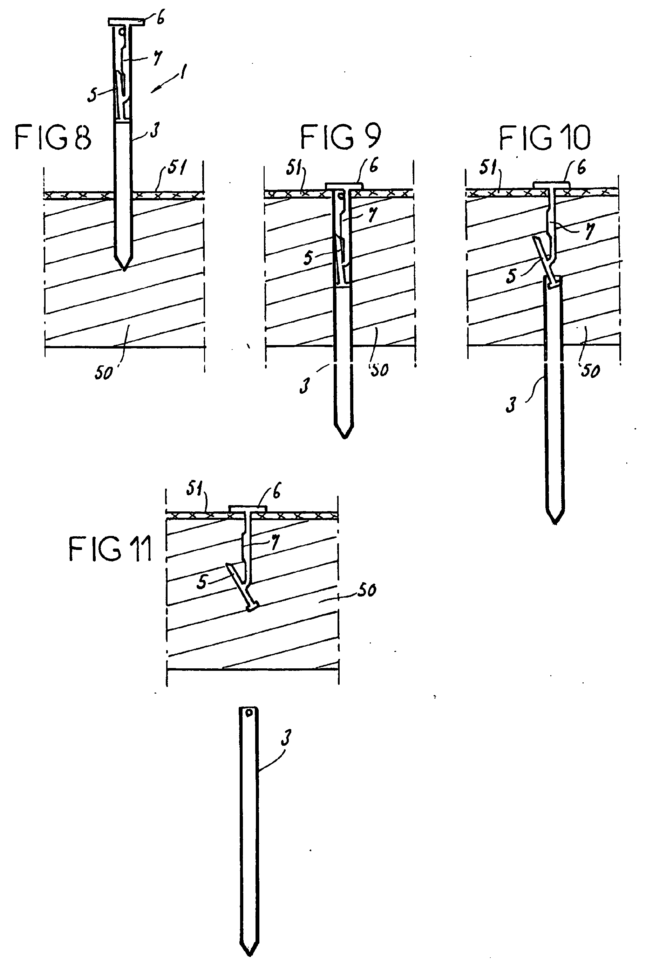

Les figures 8 à 11 montrent un ensemble 1 très similaire à celui décrit ci-dessus, sinon que l'aiguille 3 est rectiligne et qu'elle est engagée au travers du renfort 51 et de la paroi 50, le barreau 5 étant ancré à l'intérieur de cette paroi 50 une fois qu'il est séparé de l'aiguille 3.

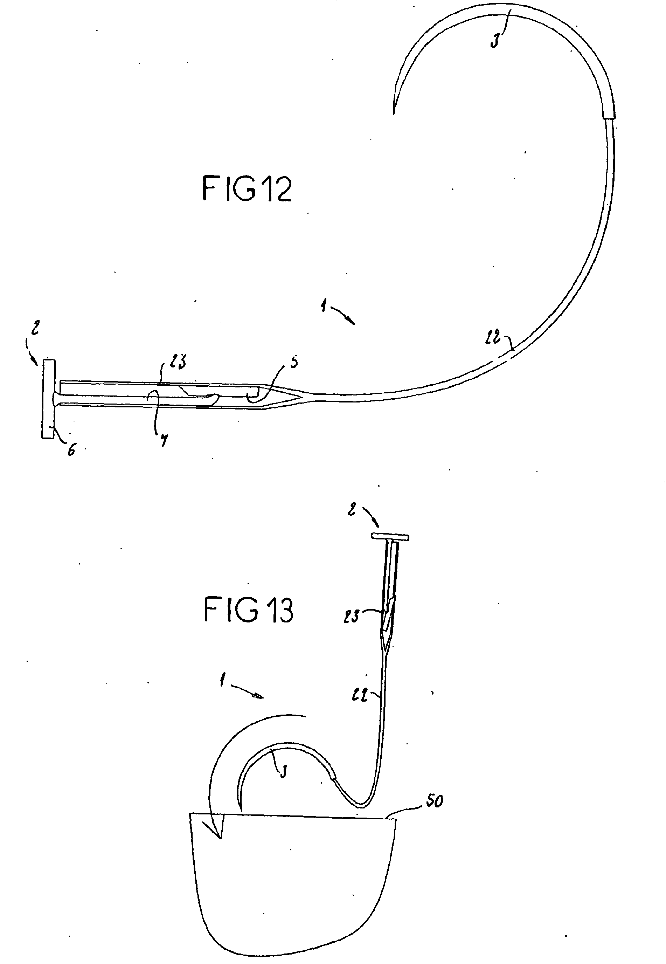

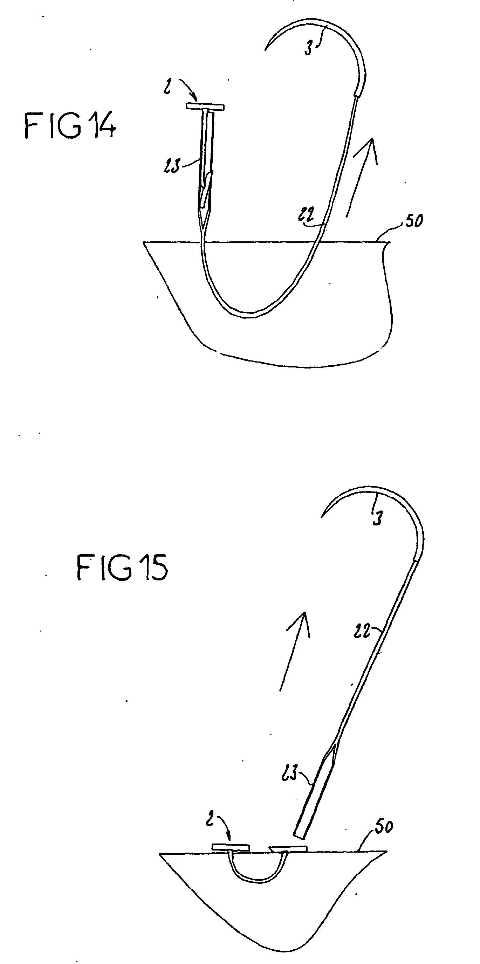

Les figures 12 à 14 montrent des variantes de réalisation de l'attache 2 et une deuxième forme de réalisation de l'ensemble 1. Les éléments déjà décrits, qui se retrouvent de manière identique ou similaire sur ces figures, sont désignés par les mêmes références numériques.

La figure 12 montre un ensemble 1 dans lequel l'aiguille 3 est une aiguille de suture classique, c'est-à-dire comprenant une extrémité proximale propre à être sertie sur un fil de suture.

Dans ce cas, l'ensemble 1 comprend une partie filiforme souple 22 dont une extrémité est reliée à l'extrémité proximale de l'aiguille 3 par sertissage et dont l'autre extrémité est raccordée à un manchon 23 recevant en lui le barreau d'arrêt 5 et de la partie filiforme 7 de l'attache 2.

La partie 22 et le manchon 23 sont formés à partir d'un tube en matériau thermorétractable, notamment en polytétrafluoroéthylène (PTFE), une thermorétraction de ce matériau étant réalisée sur la partie de ce tube destinée à constituer la partie 22. La liaison amovible entre le manchon 23 et l'attache 2 est obtenue par un choix judicieux du diamètre du tube en matériau thermorétractable, ce diamètre étant tel qu'il existe des frottements entre l'attache 2 et le manchon 23 lorsque cette attache est insérée dans ce manchon.

Comme le montrent les figures 13 à 15, la partie 22 augmente les possibilités de mobilité de l'aiguille 3 par rapport à l'attache 2, ce qui facilite la manipulation de l'aiguille. Cette partie 22 permet également que l'attache 2 soit située à distance de l'aiguille 3 lors des manipulations de cette aiguille, de manière à éliminer tout risque de séparation intempestive de l'attache 2 et de l'aiguille 3 lors de ces manipulations.

Dans le cas de l'ensemble 1 montré sur la figure 16, une ouverture latérale 24 est aménagée dans le manchon 23, et une extrémité du barreau d'arrêt 5 est engagée amoviblement dans cette fenêtre 24. La séparation de l'attache 2 et du manchon 23 peut alors être réalisée par déchirure partielle ou totale de la paroi du manchon à partir de cette fenêtre 24.

L'ensemble 1 montré sur la figure 17 comprend également une partie 22 et un manchon 23 formé au moyen d'un tube en matériau thermorétractable. Dans ce cas, un fil ruptible 25 formant une boucle autour du barreau 5 est engagé dans le tube avant de réaliser la thermorétraction. Ce fil 25 permet de réaliser la liaison amovible entre le manchon 23 et l'attache 2.

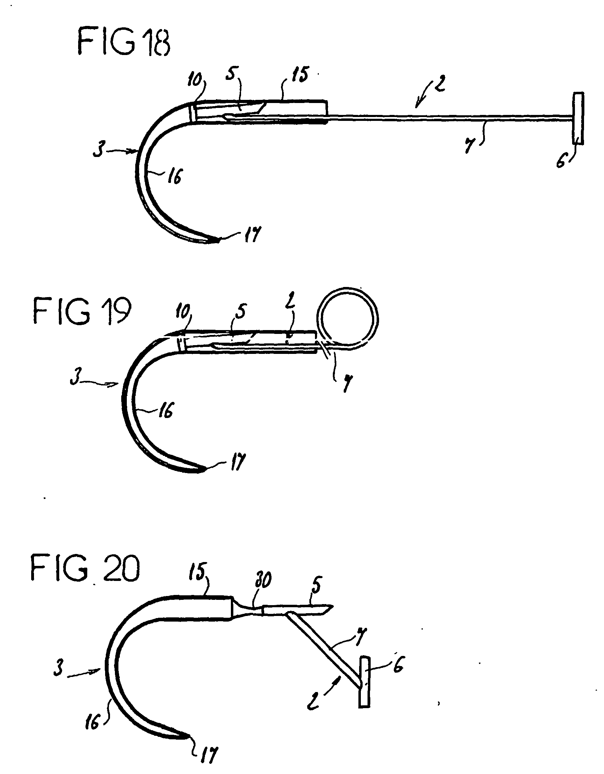

Dans le cas de la figure 18, la partie intermédiaire 7 a une longueur telle que plusieurs passages successifs de l'aiguille 3 au travers de la paroi 50 sont possibles.

Dans le cas de la figure 19, la partie intermédiaire 7 est formée par un fil propre à être utilisé pour réaliser une suture. Ce fil est enroulé sur une bobine le temps de l'introduction de l'ensemble 1 dans le corps du patient.

Dans le cas de la figure 20, l'aiguille 3 et l'attache 2 sont moulées en une seule et même pièce de matériau. L'aiguille 3 et le barreau 5 sont alors reliés l'un à l'autre par un pont de matière 30 dimensionné de façon à se rompre au-delà du seuil de force précité.

Ainsi qu'il apparaît de ce qui précède, l'invention fournit un ensemble 1 présentant de nombreux avantages par rapport aux systèmes homologues de la technique antérieure. Cet ensemble 1 a en effet pour avantage déterminant d'avoir une structure simple et un fonctionnement facile, et de présenter des possibilités d'utilisation étendues. En particulier, cet ensemble est très facile à utiliser, tant avec des techniques de coelioscopie qu'avec des techniques d'abord direct ; l'ancrage peut être réalisé par un abord de la paroi à équiper au niveau de la face de cette paroi devant recevoir l'élément prothétique, ou peut être réalisé de manière transcutanée ; l'ensemble 1 peut être conçu de manière à être à usage unique, permet de donner toutes formes appropriées à la partie distale de l'aiguille, notamment une forme courbe ou arquée, et d'utiliser des types d'attaches différentes.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées. Ainsi, l'attache 2 peut être utilisée non seulement pour fixer une pièce ou un élément prothétique, notamment un renfort pariétal 51, à une paroi corporelle 50, notamment une paroi abdominale, mais également pour relier deux parois corporelles ou deux parties anatomiques entre elles, ou pour fixer tout autre type de pièces ou d'éléments à une telle paroi ; une aiguille droite peut être utilisée pour engager l'attache au travers d'un pli formé dans un tissu ; les moyens de liaison amovible de l'attache 2 peuvent comprendre une fente aménagée dans l'extrémité proximale de l'aiguille, conférant à l'aiguille une souplesse lui permettant de retenir l'attache ; l'aiguille courbe peut être de type dit "trois huitièmes", c'est-à-dire ayant une forme correspondant sensiblement à trois huitièmes d'un cercle ; l'attache peut dépasser au-delà de l'extrémité proximale de cette aiguille.

## Revendications

1. - Ensemble (1) comprenant une attache de fixation (2) à usage médical et une aiguille (3) de mise en place de cette attache (2), l'aiguille (3) étant propre à être engagée au travers, ou dans l'épaisseur, de la ou des parois (50) destinées à être équipées de l'attache (2), et cette attache (2) présentant au moins une partie d'arrêt (5), propre à prendre appui contre une paroi (50), et une partie déformable filiforme (7) reliée à ladite partie d'arrêt (5) ; l'attache (2) est élastiquement déformable au niveau de la liaison entre ladite partie d'arrêt (5) et ladite partie déformable filiforme (7) de telle sorte que la partie d'arrêt (5) est mobile par rapport à la partie déformable filiforme (7) entre une position de déformation élastique de ladite liaison, dans laquelle la partie d'arrêt (5) et la partie déformable filiforme (7) peuvent être substantiellement inscrites à l'intérieur de la section transversale de l'aiguille (3), et une position de non déformation, dans laquelle la partie d'arrêt (5) est à même de prendre appui contre la ou les parois (50) et d'assurer l'ancrage de l'attache (2) ;
ensemble (1) **caractérisé en ce que** l'aiguille (3) et l'attache (2) comprennent des moyens (3, 10 ; 30) de liaison amovible de la partie d'arrêt (5) à la partie proximale (15) de l'aiguille (3), cette liaison étant suffisamment solide pour que la partie d'arrêt (5) reste solidaire de l'aiguille (3) lors des manipulations de l'ensemble (1) au moment de la saisie de cet ensemble (1), de l'introduction de celui-ci dans le corps du patient et de l'insertion de l'aiguille (3) dans ladite paroi (50), mais étant libérable lorsqu'une traction est exercée sur l'aiguille (3), dans le sens d'insertion de l'aiguille, au-delà d'un seuil de force supérieur aux contraintes exercées sur l'attache (2) par lesdites manipulations, la libération de l'attache permettant, par rappel élastique de ladite liaison, d'amener ladite partie d'arrêt (5) en position de non déformation, ladite aiguille (3) présentant une cavité intérieure (20) débouchant dans sa partie proximale (15), et recevant ladite partie d'arrêt (5) de l'attache (2) dans cette cavité (20) ; lesdits moyens de liaison amovible comprenant alors :
- la paroi de l'aiguille- (3) délimitant ladite cavité (20) et la partie d'arrêt (5) de l'attache (2), les dimensions de ladite cavité (20) et de ladite partie d'arrêt (5) étant déterminées de telle sorte qu'il existe des frottements entre cette partie d'arrêt (5) et cette paroi de l'aiguille (3), et/ou
- un ou plusieurs bossages (25) faisant saillie à l'intérieur de la cavité (20), propres à constituer un "point dur" devant être franchi par ladite partie d'arrêt (5) pour permettre de libérer l'attache (2).

2. - Ensemble (1) selon la revendication 1, **caractérisé en ce que** chaque bossage (25) est formé par un embouti aménagé dans la zone d'extrémité proximale de l'aiguille (3).

3. - Ensemble (1) selon la revendication 1 ou la revendication 2, **caractérisé en ce que** :
- l'aiguille (3) présente une cavité (20) proximale ayant un diamètre constant sur une portion proximale destinée à recevoir l'attache (2) et présentant une réduction progressive de ce diamètre sur une portion distale ;
- l'attache (2) comprend un barreau distal (5), un barreau proximal (6) et une partie intermédiaire (7) reliant ces barreaux (5, 6) l'un à l'autre ; le barreau distal (5) présente une collerette (10) dont le diamètre est légèrement inférieur au diamètre de ladite portion proximale de la cavité (20), de sorte que cette collerette (10) peut être introduite et peut coulisser dans cette portion proximale jusqu'au niveau de la portion distale de cette cavité (20), dans laquelle cette collerette (10) se coince ; la longueur de la partie intermédiaire (7) est telle que le barreau proximal (6) vient au contact de l'extrémité proximale (21) de l'aiguille (3) lorsque la collerette (10) se trouve en un emplacement produisant un coincement adéquat de cette collerette (10).

4. - Ensemble (1) comprenant une attache de fixation (2) à usage médical et une aiguille (3) de mise en place de cette attache (2), l'aiguille (3) étant propre à être engagée au travers, ou dans l'épaisseur, de la ou des parois (50) destinées à être équipées de l'attache (2), et cette attache (2) présentant au moins une partie d'arrêt (5), propre à prendre appui contre une paroi (50), et une partie déformable filiforme (7) reliée à ladite partie d'arrêt (5) ; l'attache (2) est élastiquement déformable au niveau de la liaison entre ladite partie d'arrêt (5) et ladite partie déformable filiforme (7) de telle sorte que la partie d'arrêt (5) est mobile par rapport à la partie déformable filiforme (7) entre une position de déformation élastique de ladite liaison, dans laquelle la partie d'arrêt (5) et la partie déformable filiforme (7) peuvent être substantiellement inscrites à l'intérieur de la section transversale de l'aiguille (3), et une position de non déformation, dans laquelle la partie d'arrêt (5) est à même de prendre appui contre la ou les parois (50) et d'assurer l'ancrage de l'attache (2) ;
ensemble (1) **caractérisé en ce que** l'aiguille (3) et l'attache (2) comprennent des moyens (3, 10 ; 30) de liaison amovible de la partie d'arrêt (5) à la partie proximale (15) de l'aiguille (3), cette liaison étant suffisamment solide pour que la partie d'arrêt (5) reste solidaire de l'aiguille (3) lors des manipulations de l'ensemble (1) au moment de la saisie de cet ensemble (1), de l'introduction de celui-ci dans le corps du patient et de l'insertion de l'aiguille (3) dans ladite paroi (50), mais étant libérable lorsqu'une traction est exercée sur l'aiguille (3), dans le sens d'insertion de l'aiguille, au-delà d'un seuil de force supérieur aux contraintes exercées sur l'attache (2) par lesdites manipulations, la libération de l'attache permettant, par rappel élastique de ladite liaison, d'amener ladite partie d'arrêt (5) en position de non déformation, ledit ensemble comprenant en outre une partie filiforme souple (22) reliée à l'extrémité proximale de l'aiguille (3) et à un manchon (23) recevant ladite partie d'arrêt (5) de l'attache (2).

5. - Ensemble (1) selon la revendication 4, **caractérisé en ce que** lesdits moyens de liaison amovible comprennent :
- les dimensions respectives du manchon (23) et de l'attache (2), ces dimensions étant telles qu'il existe des frottements entre l'attache (2) et le manchon (23) lorsque cette attache (2) est insérée dans ce manchon (23) ; et/ou
- une ouverture latérale (24) aménagée dans le manchon (23), dans laquelle est engagée amoviblement une portion de l'attache (2), en particulier une extrémité que présente ladite partie d'arrêt (5) ; et/ou
- un fil (25) présent dans le manchon (23), engagé amoviblement autour d'une partie (5, 7) de l'attache (2).

6. - Ensemble (1) selon la revendication 5, **caractérisé en ce que** le fil (25) forme une boucle autour d'une partie (5, 7) de l'attache (2) et est ruptible.

7. - Ensemble (1) comprenant une attache de fixation (2) à usage médical et une aiguille (3) de mise en place de cette attache (2), l'aiguille (3) étant propre à être engagée au travers, ou dans l'épaisseur, de la ou des parois (50) destinées à être équipées de l'attache (2), et cette attache (2) présentant au moins une partie d'arrêt (5), propre à prendre appui contre une paroi (50), et une partie déformable filiforme (7) reliée à ladite partie d'arrêt (5) ; l'attache (2) est élastiquement déformable au niveau de la liaison entre ladite partie d'arrêt (5) et ladite partie déformable filiforme (7) de telle sorte que la partie d'arrêt (5) est mobile par rapport à la partie déformable filiforme (7) entre une position de déformation élastique de ladite liaison, dans laquelle la partie d'arrêt (5) et la partie déformable filiforme (7) peuvent être substantiellement inscrites à l'intérieur de la section transversale de l'aiguille (3), et une position de non déformation, dans laquelle la partie d'arrêt (5) est à même de prendre appui contre la ou les parois (50) et d'assurer l'ancrage de l'attache (2) ;
ensemble (1) **caractérisé en ce que** l'aiguille (3) et l'attache (2) comprennent des moyens (3, 10 ; 30) de liaison amovible de la partie d'arrêt (5) à la partie proximale (15) de l'aiguille (3), cette liaison étant suffisamment solide pour que la partie d'arrêt (5) reste solidaire de l'aiguille (3) lors des manipulations de l'ensemble (1) au moment de la saisie de cet ensemble (1), de l'introduction de celui-ci dans le corps du patient et de l'insertion de l'aiguille (3) dans ladite paroi (50), mais étant libérable lorsqu'une traction est exercée sur l'aiguille (3), dans le sens d'insertion de l'aiguille, au-delà d'un seuil de force supérieur aux contraintes exercées sur l'attache (2) par lesdites manipulations, la libération de l'attache permettant, par rappel élastique de ladite liaison, d'amener ladite partie d'arrêt (5) en position de non déformation, ladite aiguille (3) et la partie d'arrêt (5) de l'attache (2) étant reliées l'une à l'autre par un pont de matière (30) dimensionné de façon à se rompre au-delà dudit seuil de force.

8. - Ensemble (1) selon la revendication 7, **caractérisé en ce que** l'aiguille (3) et l'attache (2) sont moulées en une seule et même pièce de matériau.

9. - Procédé de fabrication d'un ensemble selon l'une des revendications 4 à 6, **caractérisé en ce qu'**il comprend les étapes consistant à :
- utiliser un tube en matériau thermorétractable, notamment en polytétrafluoroéthylène (PTFE), et
- réaliser une thermorétraction de ce matériau sur la majeure partie de ce tube, pour constituer ladite partie filiforme souple (22), sans réaliser de thermorétraction sur le reste de ce tube, destiné à constituer ledit manchon (23).

10. - Procédé selon la revendication 9, de fabrication d'un ensemble selon la revendication 5 ou la revendication 6, **caractérisé en ce qu'**il comprend l'étape consistant à engager le fil (25) dans le tube avant de réaliser la thermorétraction.

## Claims

1. Kit (1) comprising a medical fixing element (2) and a needle (3) for placing this fixing element (2), the needle (3) being able to be engaged through, or within the thickness of, the wall or walls (50) intended to be fitted with the fixing element (2), and this fixing element (2) having at least one stop part (5), able to bear against a wall (50), and a filiform deformable part (7) joined to said stop part (5); the fixing element (2) is elastically deformable at the area of the connection between said stop part (5) and said filiform deformable part (7) such that the stop part (5) is movable relative to the filiform deformable part (7) between a position of elastic deformation of said connection, in which the stop part (5) and the filiform deformable part (7) can be substantially inscribed within the cross section of the needle (3), and a position of nondeformation, in which the stop part (5) is able to bear against the wall or walls (50) and ensure anchoring of the fixing element (2);
the kit (1) being **characterized in that** the needle (3) and the fixing element (2) comprise means (3, 10; 30) for detachable connection of the stop part (5) to the proximal part (15) of the needle (3), this connection being sufficiently robust to ensure that the stop part (5) remains integral with the needle (3) during maneuvers of the kit (1) when taking hold of this kit (1), introducing it into the body of the patient, and inserting the needle (3) into said wall (50), but being releasable when a traction is exerted on the needle (3), in the direction of insertion of the needle, beyond a force threshold greater than the stresses exerted on the fixing element (2) by said maneuvers, the release of the fixing element making it possible to bring said stop part (5) to the position of nondeformation, by means of elastic return of said connection, said needle (3) having an inner cavity (20) opening out in its proximal part (15) and receiving said stop part (5) of the fixing element (2) in this cavity (20); said means for detachable connection then comprising:
- the wall of the needle (3) delimiting said cavity (20) and the stop part (5) of the fixing element (2), the dimensions of said cavity (20) and of said stop part (5) being determined such that friction exists between this stop part (5) and this wall of the needle (3) and/or
- one or more bosses (25) projecting inside the cavity (20) and able to form a "hard point" which has to be passed by said stop part (5) in order to permit release of the fixing element (2).

2. Kit (1) according to Claim 1, **characterized in that** each boss (25) is formed by a stamped-in part in the proximal end zone of the needle (3).

3. Kit (1) according to Claim 1 or Claim 2, **characterized in that**:
- the needle (3) has a proximal cavity (20) having a constant diameter along a proximal portion intended to receive the fixing element (2) and having a progressive reduction of this diameter along a distal portion;
- the fixing element (2) comprises a distal bar (5), a proximal bar (6) and an intermediate part (7) joining these bars (5, 6) to one another; the distal bar (5) has a flange (10) whose diameter is slightly smaller than the diameter of said proximal portion of the cavity (20), so that this flange (10) can be introduced into and can slide in this proximal portion as far as the distal portion of this cavity (20) in which this flange (10) becomes wedged; the length of the intermediate part (7) is such that the proximal bar (6) comes into contact with the proximal end (21) of the needle (3) when the flange (10) is in a position producing sufficient wedging of this flange (10).

4. Kit (1) comprising a medical fixing element (2) and a needle (3) for placing this fixing element (2), the needle (3) being able to be engaged through, or within the thickness of, the wall or walls (50) intended to be fitted with the fixing element (2), and this fixing element (2) having at Least one stop part (5), able to bear against a wall (50), and a filiform deformable part (7) joined to said stop part (5); the fixing element (2) is elastically deformable at the area of the connection between said stop part (5) and said filiform deformable part (7) such that the stop part (5) is movable relative to the filiform deformable part (7) between a position of elastic deformation of said connection, in which the stop part (5) and the filiform deformable part (7) can be substantially inscribed within the cross section of the needle (3), and a position of nondeformation, in which the stop part (5) is able to bear against the wall or walls (50) and ensure anchoring of the fixing element (2);
the kit (1) being **characterized in that** the needle (3) and the fixing element (2) comprise means (3, 10; 30) for detachable connection of the stop part (5) to the proximal part (15) of the needle (3), this connection being sufficiently robust to ensure that the stop part (5) remains integral with the needle (3) during maneuvers of the kit (1) when taking hold of this kit (1), introducing it into the body of the patient, and inserting the needle (3) into said wall (50), but being releasable when a traction is exerted on the needle (3), in the direction of insertion of the needle, beyond a force threshold greater than the stresses exerted on the fixing element (2) by said maneuvers, the release of the fixing element making it possible to bring said stop part (5) to the position of nondeformation, by means of elastic return of said connection, said kit further comprising a flexible filiform part (22) joined to the proximal end of the needle (3) and to a sleeve (23) receiving said stop part (5) of the fixing element (2).

5. Kit (1) according to Claim 4, **characterized in that** said means for detachable connection comprise:
- the respective dimensions of the sleeve (23) and of the fixing element (2), these dimensions being such that friction exists between the fixing element (2) and the sleeve (23) when this fixing element (2) is inserted into this sleeve (23); and/or
- a lateral opening (24) which is formed in the sleeve (23) and in which a portion of the fixing element (2) is engaged detachably, in particular an end of said stop part (5); and/or
- a thread (25) present in the sleeve (23) and engaged detachably around a part (5, 7) of the fixing element (2).

6. Kit (1) according to Claim 5, **characterized in that** the thread (25) forms a loop around a part (5, 7) of the fixing element (2) and is breakable.

7. Kit (1) comprising a medical fixing element (2) and a needle (3) for placing this fixing element (2), the needle (3) being able to be engaged through, or within the thickness of, the wall or walls (50) intended to be fitted with the fixing element (2), and this fixing element (2) having at least one stop part (5), able to bear against a wall (50), and a filiform deformable part (7) joined to said stop part (5); the fixing element (2) is elastically deformable at the area of the connection between said stop part (5) and said filiform deformable part (7) such that the stop part (5) is movable relative to the filiform deformable part (7) between a position of elastic deformation of said connection, in which the stop part (5) and the filiform deformable part (7) can be substantially inscribed within the cross section of the needle (3), and a position of nondeformation, in which the stop part (5) is able to bear against the wall or walls (50) and ensure anchoring of the fixing element (2); the kit (1) being **characterized in that** the needle (3) and the fixing element (2) comprise means (3, 10; 30) for detachable connection of the stop part (5) to the proximal part (15) of the needle (3), this connection being sufficiently robust to ensure that the stop part (5) remains integral with the needle (3) during maneuvers of the kit (1) when taking hold of this kit (1), introducing it into the body of the patient, and inserting the needle (3) into said wall (50), but being releasable when a traction is exerted on the needle (3), in the direction of insertion of the needle, beyond a force threshold greater than the stresses exerted on the fixing element (2) by said maneuvers, the release of the fixing element making it possible to bring said stop part (5) to the position of nondeformation, by means of elastic return of said connection, said needle (3) and the stop part (5) of the fixing element (2) being joined to one another by a material bridge (30) dimensioned so as to rupture beyond said force threshold.

8. Kit (1) according to Claim 7, **characterized in that** the needle (3) and the fixing element (2) are molded in one and the same piece of material.

9. Method for producing a kit according to one of Claims 4 to 6, **characterized in that** it comprises the steps of:
- using a tube of heat-shrinkable material, in particular polytetrafluoroethylene (PTFE), and
- heat-shrinking this material along the greater part of this tube in order to form said flexible filiform part (22), without heat-shrinking the rest of this tube intended to form said sleeve (23).

10. Method according to Claim 9 for producing a kit according to Claim 5 or Claim 6, **characterized in that** it comprises the step of engaging the thread (25) in the tube before heat-shrinking.

## Patentansprüche

1. Satz (1) mit einem Befestigungselement (2) für medizinischer Gebrauch und einer Nadel (3) zur Anordnung dieses Befestigungungselements (2), wobei die Nadel (3) dafür vorgesehen ist, durch die oder in die Dicke der Wand oder Wände (50) eingeführt zu werden, die mit dem Befestigungselement (2) versehen werden sollen, und dieses Befestigungselement (2) zumindest einen Arretierteil (5) aufweist, der dafür vorgesehen ist, sich gegen eine Wand (50) abzustützen, und einen verformbaren drahtförmigen Teil (7), der mit dem Arretierteil (5) verbunden ist; das Befestigungselement (2) ist im Bereich der Verbindung zwischen dem Arretierteil (5) und dem verformbarer drahtförmigen Teil (7) elastisch verformbar, so dass der Arretierteil (5) relativ zu dem verformbaren drahtförmigen Teil (7) beweglich ist zwischen einer elastisch verformten Position der Verbindung, in welcher der Arretierteil (5) und der verformbare drahtförmige Teil (7) im Wesentlichen in das Innere des Querschnitts der Nadel (3) eingetreten sein können, und einer nicht verformten Position, in welcher der Arretierteil (5) imstande ist, sich gegen die Wand oder Wände (50) abzustützen und die Verankerung des Befestigungselements (2) zu bewirken; wobei der Satz (1) **dadurch gekennzeichnet ist, dass** die Nadel (3) und das Befestigungselement (2) Mittel (3, 10; 30) zur lösbaren Verbindung des Arretierteils (5) mit cem proximalen Teil (15) der Nadel (3) enthalten, wobei diese Verbindung ausreichend fest ist, so dass der Arretierteil (5) bei der Handhabung des Satzes (1) zum Zeitpunkt des Ergreifens des Satzes (1), von dessen Einführung in den Körper das Patienten und des Eintritts der Nadel in die Wand (50) fest mit der Nadel (3) verbunden bleibt, jedoch lösbar ist, sobald in der Einführungsrichtung der Nadel ein Zug auf die Nadel (3) jenseits eines Kraftschwellwertes ausgeübt wird, der höher als die durch diese Handhabung auf das Befestigungselement (2) ausgeübten Kräfte ist, wobei die Freigabe des Befestigungselementes es durch elastischen Rückzug der Verbindung gestattet, den Arretierteil (5) in die nichtverformte Position zu bringen, wobei die Nadel (3) einen inneren Hohlraum (20) aufweist, der in ihrem proximalen Teil (15) ausmündet und den Arretierteil (5) des Befestigungselements (2) in diesem Hohlraum (20) aufnimmt; wobei die Mittel für die lösbare Verbindung sodann enthalten:
• die Wand der Nadel (3), die den Hohlraum (20) und den Arretierteil (5) des Befestigungselements (2) begrenzt, wobei die Abmessungen des Hohlraums (20) und des Arretierteils (5) derart bestimmt sind, dass Reibungen zwischen dem Arretierteil (5) und der Wand der Nadel (3) vorhanden sind, und/oder
• einen oder mehrere Höcker (25), die in das Innere des Hohlraums (20) vorspringen, die derart vorgesehen sind, dass sie einen "harten Punkt" bilden, der von dem Arretierteil (5) überwunden werden muss, um das Befestigungselement (2) freizugeben.

2. Satz (1) nach Anspruch 1, **dadurch gekennzeichnet, dass** jeder Höcker (25) durch eine Ausbuchtung geformt ist, die in dem proximalen Endbereich der Nadel (3) vorgesehen ist.

3. Satz (1) nach Anspruch 1 oder Anspruch 2, **dadurch gekennzeichnet, dass**:
• die Nadel (3) einen proximalen Hohlraum (20) aufweist, der einen Durchmesser hat, der über einen proximalen, zur Aufnahme des Befestigungselements (2) vorgesehenen abschnitt konstant ist, und an einem distalen Abschnitt eine progressive Reduzierung des Durchmessers aufweist;
• das Befestigungselement (2) einen distalen Stab (5), einen proximalen Stab (6) und einen mittleren Teil (7) aufweist, der die beiden Stäbe (5, 6) miteinander verbindet; der distale Stab (5) einen Kragen (10) aufweist, dessen Durchmesser geringfügig kleiner als der Durchmesser des proximalen Bereichs des Hohlraums (20) ist, so dass der Kragen (10) in diesen proximalen Bereich eintreten kann und bis zum Bereich des distalen Bereichs des Hohlraums (20) gleiten kann, in dem sich der Kragen (10) verklemmt; die Länge des mittleren Teils (7) derart beschaffen ist, dass der proximale Stab (6) in Kontakt mit dem proximalen Ende (21) der Nadel (3) kommt, wenn sich der Kragen (10) an einer Stelle befindet, die ein entsprechendes Verklemmen des Kragens (10) bewirkt.

4. Satz (1) mit einem Befestigungselement (2) für medizinischen Gebrauch und einer Nadel (3) zur Anordnung dieses Befestigungselements (2), wobei die Nadel (3) dafür vorgesehen ist, durch die oder in die Dicke der Wand oder Wände (50) eingeführt zu werden, die mit dem Befestigungselement (2) versehen werden sollen, und dieses Befestigungselement (2) zumindest einen Arretierteil (5) aufweist, der dafür vorgesehen ist, sich gegen eine Wand (50) abzustützen, und einen verformbaren drahtförmigen Teil (7), der mit dem Arretierteil (5) verbunden ist; das Befestigungselement (2) ist im Bereich der Verbindung zwischen dem Arretierteil (5) und dem verformbarer drahtförmigen Teil (7) elastisch verformbar, so dass der Arretierteil (5) relativ zu dem verformbaren drahtförmigen Teil (7) beweglich ist zwischen einer elastisch verformten Position der Verbindung, in welcher der Arretierteil (5) und der verformbare drahtförmige Teil (7) im Wesentlichen in das Innere des Querschnitts der Nadel (3) eingetreten sein können, und einer nicht verformten Position, in welcher der Arretierteil (5) imstande ist, sich gegen die Wand oder Wände (50) abzustützen und die Verankerung des Befestigungselements (2) zu bewirken; wobei der Satz (1) **dadurch gekennzeichnet ist, dass** die Nadel (3) und das Befestigungselement (2) Mittel (3, 10; 30) zur lösbaren Verbindung des Arretierteils (5) mit dem proximalen Teil (15) der Nadel (3) enthalten, wobei diese Verbindung ausreichend fest ist, so dass der Arretierteil (5) bei der Handhabung des Satzes (1) zum Zeitpunkt des Ergreifens des Satzes (1), von dessen Einführung in den Körper des Patienten und des Eintritts der Nadel in die Wand (50) fest mit der Nadel (3) verbunden bleibt, jedoch lösbar ist, sobald in der Einführungsrichtung der Nadel ein Zug auf die Nadel (3) jenseits eines Kraftschwellwertes ausgeübt wird, der höher als die durch diese Handhabung auf das Befestigungselement (2) ausgeübten Kräfte ist, wobei die Freigabe des Befestigungselementes es durch elastischen Rückzug der Verbindung gestattet, den Arretierteil (5) in die nichtverformte Position zu bringen, wobei der Satz ferner einen biegsamer drahtförmigen Teil (22) aufweist, der mit dem proximalen Ende der Nadel (3) und einer Hülse (23) zur Aufnahme des Arretierteils (5) des Befestigungselements (2) verbunden ist.

5. Satz (1) nach Anspruch 4, **dadurch gekennzeichnet, dass** die Mittel zur lösbaren Verbindung aufweisen:
• die jeweiligen Abmessungen der Hülse (23) und des Befestigungselements (2), wobei diese Abmessungen derart beschaffen sind, dass Reibungen zwischen dem Befestigungselement (2) und der Hülse (23) vorhanden sind, wenn das Befestigungselement (2) in die Hülse (23) eingetreten ist; und/oder
• eine seitliche Öffnung (24), die in der Hülse (23) vorgesehen ist, in die ein Abschnitt des Befestigungselements (2), insbesondere ein Ende, welches der Arretierteil (5) aufweist, lösbar eingeführt ist, und/oder
• einen Draht (25), der in der Hülse (23) vorhanden ist, der lösbar um einen Teil (5; 7) des Befestigungselements (2) herum eingeführt ist.

6. Satz (1) nach Anspruch 5, **dadurch gekennzeichnet, dass** der Draht. (25) eine Schleife um einen Teil (5, 7) des Befestigungselements (2) formt und brechbar ist.

7. Satz: (1) mit einem Befestigungselement (2) für medizinischer Gebrauch und einer Nadel (3) zur Anordnung dieses Befestigungselements (2), wobei die Nadel (3) dafür vorgesehen ist, durch die oder in die Dicke der Wand oder Wände (50) eingeführt zu werden, die mit dem Befestigungselement (2) versehen werden sollen, und dieses Befestigungselement (2) zumindest, einen Arretierteil (5) aufweist, der dafür vorgesehen ist, sich gegen eine Wand (50) abzustützen, und einen verformbaren drahtförmigen Teil (7), der mit dem Arretierteil (5) verbunden ist; das Befestigungselement (2) ist im Bereich der Verbindung zwischen dem Arretierteil (5) und dem verformbaren drahtförmigen Teil (7) elastisch verformbar, so dass der Arretierteil (5) relativ zu dem verformbaren drahtförmigen Teil (7) beweglich ist zwischen einer elastisch verformten Position der Verbindung, in welcher der Arretierteil (5) und der verformbare drahtförmige Teil (7) im Wesentlichen in das Innere des Querschnitts der Nadel (3) eingetreten sein können, und einer nicht verformten Position, in welcher der Arretierteil (5) imstande ist, sich gegen die Wand oder Wände (50) abzustützen und die Verankerung des Befestigungselements (2) zu bewirken; wobei der Satz (1) **dadurch gekennzeichnet ist, dass** die Nadel (3) und das Befestigungselement (2) Mittel (3, 10; 30) zur lösbaren Verbindung des Arretierteils (5) mit dem proximalen Teil (15) der Nadel (3) enthalten, wobei diese Verbindung ausreichend fest ist, so dass der Arretierteil (5) bei der Handhabung des Satzes (1) zum Zeitpunkt des Ergreifens des Satzes (1), von dessen Einführung in den Körper des Patienten und des Eintritts der Nadel in die Wand (50) fest mit der Nadel (3) verbunden bleibt, jedoch lösbar ist, sobald in der Einführungsrichtung der Nadel ein Zug auf die Nadel (3) jenseits eines Kraftschwellwertes ausgeübt wird, der höher als die durch diese Handhabung auf das Befestigungselement (2) ausgeübten Kräfte ist, wobei die Freigabe des Befestigungselementes es durch elastischen Rückzug der Verbindung gestattet, den Arretierteil (5) in die nichtverformte Position zu bringen, wobei die Nadel (3) und der Arretierteil (5) des Befestigungselements (2) über eine Materialbrücke (30) miteinander verbunden sind, die derart dimensioniert ist, dass sie jenseits des Kraftschwellwerts bricht.

8. Satz (1) nach Anspruch 7, **dadurch gekennzeichnet, dass** die Nadel (3) und das Befestigungselement (2) einstückig aus demselben Material gegossen sind.

9. Verfahren zur Herstellung eines Satzes nach einem der Ansprüche 4 bis 6, **dadurch gekennzeichnet, dass** es die folgenden Schritte umfasst :
• - Verwendung eines Schlauches aus wärmeschrumpfendem Material, insbesondere aus Polytetrafluorethylen (PTFE) und
• - an dem größeren Teil des Schlauchs eine Wärmeschrumpfung dieses Materials durchzuführen, um den biegsamen drahtförmigen Teil (22) zu bilden, ohne an dem Rest des Schlauchs, der für die Bildung der Hülse (23) vorgesehen ist, eine Wärmeschrumpfung durchzuführen.

10. Verfahren nach Anspruch 9 zur Herstellung eines Satzes nach Anspruch 5 oder Anspruch 6, **dadurch gekennzeichnet, dass** es den Schritt enthält, der darin besteht, den Draht (25) in den Schlauch einzuführen, bevor die wärmeschrumpfung durchgeführt wird.
